# EUROPEAN PATENT APPLICATION

(11) **EP 0 972 506 A2**
(43) Date of publication of application: **19.01.2000**
(21) Application number: 99113342.2
(22) Date of filing: 09.07.1999
(51) Int. Cl.: A61J 1/10

(54) **Flexible bag for containing at least two separate substances to be mixed for medical use, and relative fabrication method**

(30) Priority: 10.07.1998 IT TO980604
(71) Applicant: Haemotronic Advanced Medical Technologies S.p.A., 41037 Mirandola (IT)
(72) Inventor: Ravizza, Renato, 30174 Verona (IT)
(74) Representative: Eccetto, Mauro

(57) **Abstract**

The bag (11) is defined by two superimposed sheets (23) of plastic material - in turn defined by a multilayer film - connected to each other along the perimeter (26), and has two chambers (12, 13) for two respective substances which are kept separate by an intermediate portion (14) at which the sheets (23) adhere to each other. Which adhesion is achieved by a cross-linking process consisting substantially in electron bombardment to cross-link the various polymer chains of the material from which the bag is made.

In actual use, the two chambers (12, 13) are connected to each other by exerting sufficient external pressure to overcome the adhesive strength of the intermediate portion (14) at which the sheets (23) contact.

The fabrication method includes the step of electron-bombarding the film (19) from which the sheets (23) are made.

## Description

The present invention relates to a flexible bag for containing at least two substances to be mixed for medical use; and to a relative fabrication method.

As is known, various medicinal substances are supplied separately and mixed when used. In particular, one of the substances to be mixed is normally defined by a liquid in which the other substance is either dissolved or diluted, e.g. for infusion into a patient's body.

Various types of bags made of flexible plastic material are known, in which the bag is defined by two separate chambers to keep the two substances separate. In one such type of bag, the two chambers are separated by a continuous sealed line in which is inserted a connecting conduit closed by a break-open valve, which, when the substances are used, is broken to enable the substances in the chambers to mix. In this type of bag, the valve is difficult to break open completely, breakage of the valve often involves repeated handling of the bag, and the valve may even be only opened partially, in which case, mixing of the substances is much slower or even incomplete, thus resulting in improper concentrations and possibly also in failure of the treatment.

In another type of bag, the chambers are separated by temporary or lightly sealed lines which are unsealed by exerting pressure on one of the two chambers to mix the substances. A major drawback of this type of bag lies in fragments of the temporary seal often remaining inside the mixture, thus seriously endangering the patient.

Finally, another type of bag has been proposed in which the two chambers are kept separate by two folds for choking an intermediate portion, with no sealing. The bag is produced by folding it into a Z shape, so as to keep the intermediate portion between the two chambers of the two substances pressed, and by covering the bag with an overbag to keep the bag firmly in the Z-shaped configuration pending use. This type of bag is fairly complex as regards filling the chambers and subsequently inserting the bag inside the overbag, by requiring delicate handling to prevent premature mixing of the two substances.

It is an object of the present invention to provide an extremely straightforward, low-cost flexible bag for containing at least two substances to be mixed, and which provides for eliminating the aforementioned drawbacks typically associated with known bags.

According to the present invention, there is provided a flexible bag for containing at least two substances to be mixed for medical use; at least one of said substances being defined by a liquid; the bag comprising two superimposed sheets of plastic material connected to each other along the perimeter to define at least two chambers for containing said substances and separated from each other by an intermediate portion, and at least two closable fittings connecting respective said chambers to the outside; said substances being mixed by connecting said chambers by means of said intermediate portion;
said bag being characterized in that said sheets are defined by multilayer films of plastic material of various polymer chains; and in that said sheets have, at said intermediate portion, an adhesive strength determined by cross-linking the respective polymer chains, and such as to normally keep the substances in said chambers separate.

The bag is produced by a method in accordance with the invention, and which comprises the steps of:
- providing a film of multilayer plastic material of various polymer chains;
- subjecting two superimposed sheets of said film to electron bombardment such as to cross-link the various polymer chains and cause adhesion of facing surfaces of the superimposed said sheets;
- sealing the two said sheets along at least part of the perimeter to form at least two chambers of said bag, and providing, for each of the chambers, at least one fitting for connection to the outside; and
- filling said chambers with said substances through said fittings, so as to leave an intermediate portion separating said chambers and defined by said adhesion.

A preferred, non-limiting embodiment of the invention will be described by way of example with reference to the accompanying drawings, in which:
Figures 1-4 show respective steps in the method of producing the bag according to the invention;
Figure 5 shows a longitudinal section of the filled bag;
Figures 6 and 7 show two variations of a detail of the bag;
Figure 8 shows a longitudinal section of the bag with a first variation of the final package;
Figure 9 shows a partial front view of the Figure 8 package with a further variation of a detail of the bag;
Figure 10 shows a longitudinal section of the bag with a second variation of the final package;
Figure 11 shows the step of mixing the substances in the bag.

As is known, a transparent plastic material may be defined by various polymer chains and coextruded into a multilayer film of predetermined thickness so as to flexible; and, when subjected to so-called cross-linking, the film varies its chemical and physical characteristics, assumes adhesive characteristics, and increases in both mechanical strength and thermal resistance.

Cross-linking substantially consists in electron-bombarding the film to cross-link the various polymer chains of the material. Though all the changes produced in the plastic material by electron bombardment have not yet been determined accurately, the most likely assumption is that the radiated electrons, on impact with the polymer chains, generate highly unstable free radicals which tend to react strongly with one another and with the nearby atoms. This results directly in greater adhesion or stickiness of the treated material, especially between two superimposed sheets of the same material, as well as in greater thermal resistance, to the extent of withstanding temperatures of well over 100°C and even of about 120°C.

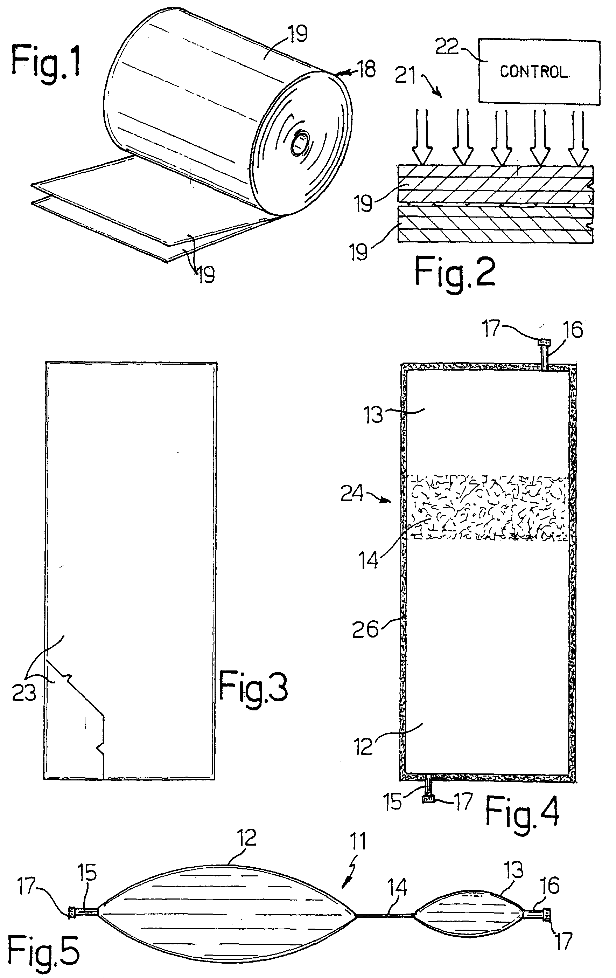

The method of producing the bag according to the invention is substantially based on exploiting the above cross-linking phenomenon of plastic materials. With reference to Figure 5, number 11 indicates as a whole a flexible bag for containing at least two substances to be mixed for medical use. At least one of the two substances is normally defined by a liquid, e.g. distilled water, whereas the other/s may each be defined by a liquid or a powder. To achieve the desired therapeutic and/or diagnostic effect, the substances must only be mixed when administered to the patient.

Figures 4 and 5 show a bag 11 for two substances to be mixed, and which are contained in two respective chambers 12 and 13. Chambers 12 and 13 are separated by an intermediate portion 14, and each have at least one respective fitting or tube 15, 16 by which to fill chamber 12, 13 with the respective substance. Each fitting 15, 16 has a removable closing member 17; and one of the two fittings, e.g. fitting 15, may also be used for withdrawing the mixture.

The method of producing bag 11 commences with a roll 18 (Figure 1) of film 19 of transparent plastic material substantially defined by various polymer chains and coextruded into a multilayer film of predetermined thickness. Roll 18 comprises two superimposed films 19 (Figure 1) which are formed in known manner by a coextrusion process whereby a tube of plastic material is formed by means of a so-called "coextrusion bubble mold", and is then folded, cut along the edges and wound into roll 18. Roll 18 is advantageously marketed by CRYOVAC under product number M312, and substantially comprises the following polymers: EPC, SEBS, LLPE, PET.

According to the method of the invention, the material, during the production process and immediately following the coextrusion stage, is transferred to a station 21 (Figure 2) where the two films 19 are subjected to cross-linking, which substantially comprises electron bombardment to cross-link the various polymer chains and produce appropriate adhesion characteristics of the two superimposed films 19.

Station 21 comprises control means 22 for appropriately regulating electron radiation to achieve a predetermined adhesion of the opposite surfaces of films 19; and the radiation also generates in the material a thermal resistance capable of withstanding sterilization temperatures of well over 100°C.

Two sheets 23 of the same size (Figure 3) and adhering perfectly to each other are then cut from the two films 19 treated as described above; respective fittings 15 and 16 (Figure 4) are inserted between the two sheets 23; and, at a sealing station 24, the two sheets 23 are heat-sealed electrically in known manner along the perimeter 26, together with respective fittings 15, 16.

As the substances are fed, at controlled pressure, through respective fittings 15 and 16 into respective chambers 12 and 13, the facing surfaces of sheets 23 are gradually parted as the force of adhesion holding the surfaces firmly in contact with each other is exceeded. Once the chambers are filled, the facing surfaces of intermediate portion 14 still adhere firmly to each other, thus separating the substances in opposite chambers 12, 13, as shown in Figures 4 and 5.

The bag configuration described so far obviously provides for perfectly separating chambers 12 and 13 pending mixing of the respective substances, as well as for thorough mixing of the substances by virtue of the maximum possible size of the passage between the chambers. Equally obvious is the possibility of the substances being mixed accidentally if the bag is not handled properly. That is, portion 14 has two very long edges extending crosswise from one side to the opposite side of the bag, and which must withstand the pressure generated by the respective substances and which tends to part the facing surfaces of sheets 23.

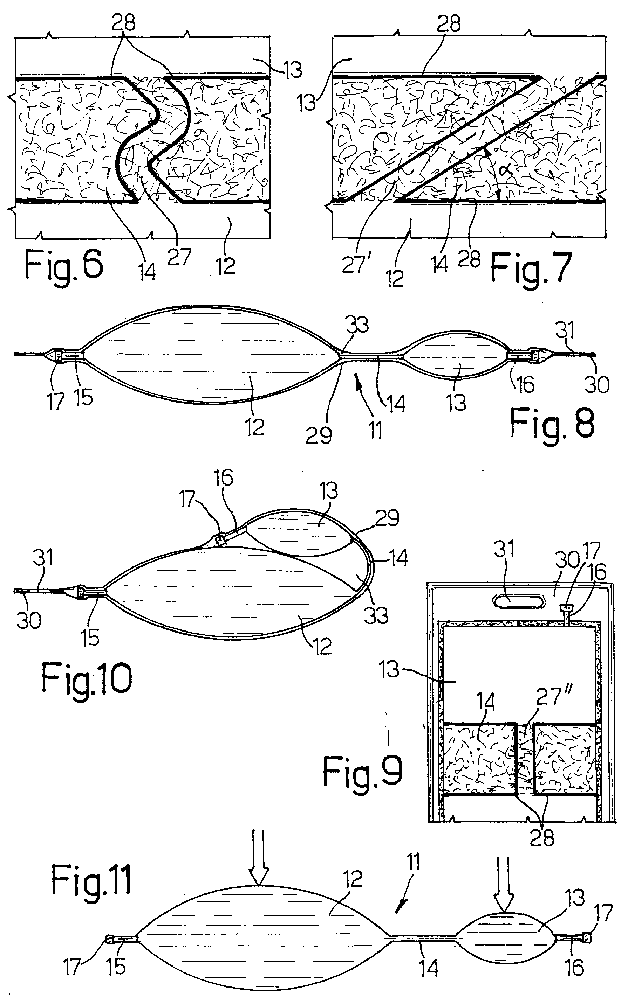

To ensure the facing surfaces can only be parted by exerting strong external pressure, a number of variations (Figures 6, 7, 9) have been provided to restrict flow of the substances, at the mixing stage, along privileged "channels" formed in portion 14 and indicated 27, 27', 27'' respectively. The channels are formed by means of seals 28 along portions of portion 14, and which provide for withstanding most of the stress and so reducing said edges.

This provides for improving resistance to occasional mechanical stress while at the same time preserving the advantages of direct communication between chambers 12 and 13 by simply parting the facing surfaces of the channels in portion 14. The seals may advantageously be formed at the same station 24 as for sealing perimeter 26.

With reference to Figures 6, 7, 9, channels 27, 27', 27'' are respectively curved, or straight at an angle α of about 30° with respect to the edges of portion 14, or simply straight and perpendicular to said edges.

Once filled, bag 11 is fitted with an outer covering 29 (Figures 8-10) made from a sheet of nonadhering material, e.g. again plastic material, enabling sterilization of bag 11 already covered.

Covering 29 is applied without choke-folding portion 14, which is no longer necessary by virtue of adhering portion 14 being perfectly sealed; and a vacuum is formed in a gap 33 between covering 29 and bag 11 to withstand, as far as possible, any accidental mechanical stress which might result in accidental communication of chambers 12 and 13. In a first solution, covering 29 (Figures 8 and 9) is applied to bag 11 with chambers 12, 13 and portion 14 fully extended. In another solution, covering 29 (Figure 10) is applied to bag 11 with chambers 12 and 13 substantially side by side and with portion 14 curving slightly in between. Whichever solution is adopted, covering 29 may comprise a portion 30 having an opening 31 (Figure 9) by which to carry bag 11.

Bag 11, covered as described above, is then sterilized in an autoclave, advantageously at a temperature of about 120°C for about 30 minutes.

To administer the substances in bag 11, covering 29 is first removed and, in the case of the Figure 10 solution, chambers 12 and 13 are fully extended. Pressure is then applied to one or both of chambers 12 and 13, as shown by the arrows in Figure 11, to part the whole of portion 14 or channels 27, 27', 27'' and so mix the two substances in chambers 12 and 13; and bag 11 may conveniently be hung onto a support to infuse the resulting mixture of substances.

The advantages, as compared with the known state of the art, of the bag according to the invention will be clear from the foregoing description. In particular, no breakage of valves or seals along the intermediate portion is required. Cross-linking of the material ensures adequate adhesion of sheets 23, as well as improved thermal resistance and mechanical strength. And, finally, sealing intermediate portion 14 to define a limited passage (channels 27, 27', 27'') ensures chambers 12 and 13 are kept separate even when subjected to accidental mechanical stress resulting, for example, from improper transport.

Clearly, changes may be made to the method and bag as described herein without, however, departing from the scope of the accompanying Claims. For example, bag 11 may comprise more than two chambers separated by respective adhering-sheet portions.

Sheets 23 may also be formed from a tubular extrusion, in which case, the sheets are already connected along two sides of the perimeter, so that sealing is reduced as compared with the above solution. Finally, a fitting, separate from fill fittings 15 and 16, may be provided for withdrawing the mixture from one of chambers 12 and 13.

## Claims

1. A flexible bag for containing at least two substances to be mixed for medical use; at least one of said substances being defined by a liquid; the bag comprising two superimposed sheets (23) of plastic material connected to each other along the perimeter (26) to define at least two chambers (12, 13) for containing said substances and separated from each other by an intermediate portion (14), and at least two closable fittings (15, 16) connecting respective said chambers (12, 13) to the outside; said substances being mixed by connecting said chambers (12, 13) by means of said intermediate portion (14);
said bag being characterized in that said sheets (23) are defined by multilayer films of plastic material of various polymer chains; and in that said sheets (23) have, at said intermediate portion (14), an adhesive strength determined by cross-linking the respective polymer chains, and such as to normally keep the substances in said chambers (12, 13) separate.

2. A bag as claimed in Claim 1, characterized in that said cross-linking of said material is the consequence of electron bombardment.

3. A bag as claimed in Claim 1 or 2, characterized in that said intermediate portion (14) is sealed, leaving at least one passage (27, 27', 27'') normally kept closed solely by the adhesion of said sheets (23) and which is opened, to permit mixing of said substances, by pressure being applied on at least one of said chambers.

4. A bag as claimed in Claim 3, characterized in that said passage (27') curves across said intermediate portion (14).

5. A bag as claimed in Claim 3, characterized in that said passage (27'') slopes at a predetermined angle (α) across said intermediate portion (14).

6. A bag as claimed in one of the foregoing Claims, characterized by comprising an outer covering (29) surrounding said bag, with no choke-folding of said intermediate portion (14).

7. A bag as claimed in Claim 6, characterized in that said outer covering (29) and said bag define a gap (33) in which a vacuum is maintained.

8. A bag as claimed in Claim 6 or 7, characterized in that said outer covering (29) is applied with said chambers (12, 13) and said intermediate portion (14) substantially fully extended.

9. A bag as claimed in Claim 6 or 7, characterized in that said outer covering (29) is applied with said chambers (12, 13) positioned substantially side by side; said intermediate portion (14) assuming an arc shape.

10. A method of producing a flexible bag (11) for containing at least two substances to be mixed for medical use; at least one of said substances being defined by a liquid; said bag (11) comprising two superimposed sheets (23) of plastic material connected to each other along the perimeter (26) to define at least two chambers (12, 13) for containing said substances and separated from each other by an intermediate portion (14); and said method comprising the steps of:
- providing a film (19) of multilayer plastic material of various polymer chains;
- subjecting two superimposed sheets (23) of said film to electron bombardment such as to cross-link the various polymer chains and cause adhesion of facing surfaces of the superimposed said sheets (23);
- sealing the two said sheets (23) along at least part of the perimeter to form at least two chambers (12, 13) of said bag, and providing, for each of the chambers, at least one fitting (15, 16) for connection to the outside; and
- filling said chambers (12, 13) with said substances through said fittings, so as to leave an intermediate portion (14) separating said chambers (12, 13) and defined by said adhesion.

11. A method as claimed in Claim 10, characterized in that said bombardment is performed simultaneously on two superimposed films (19) of said material; said sheets (23) being cut simultaneously from said films (19).

12. A method as claimed in Claim 10 or 11, characterized by appropriately calibrating the electron radiation dosage of said bombardment to obtain sufficient adhesion of said intermediate portion (14) to normally keep the substances in said chambers (12, 13) separate.

13. A method as claimed in one of Claims 10 to 12, characterized in that, prior to said filling step, said intermediate portion (14) is sealed, leaving, to permit mixing of said substances, at least one passage (27, 27', 27'') closed solely by the adhesion of said sheets (23).

14. A method as claimed in one of Claims 10 to 13, characterized by subjecting said bag (11) to sterilization in an autoclave for a given period of time at a temperature of at least approximately 100°C.

15. A method as claimed in one of Claims 10 to 14, characterized in that, after said filling step, said bag (11) is enclosed in an outer covering (29), with no choke-folding of said intermediate portion (14).

16. A method as claimed in Claim 15, characterized in that a vacuum is formed between said outer covering (29) and said bag.
